# EUROPEAN PATENT SPECIFICATION

(11) **EP 1 517 659 B1**
(45) Date of publication and mention of the grant of the patent: **14.11.2007**
(21) Application number: 03712467.4
(22) Date of filing: 14.04.2003
(51) Int. Cl.: A61F 13/476, A61F 13/472

(54) **THONG SANITARY NAPKIN WITH AN IMPROVED FASTENING SYSTEM**
EINLAGE FÜR TANGASLIP MIT VERBESSERTEM BEFESTIGUNGSSYSTEM
SERVIETTE HYGIENIQUE A LANIERE DOTEE D'UN SYSTEME D'ATTACHE AMELIORE

(30) Priority: 01.07.2002 GR 2002100315
(43) Date of publication of application: 30.03.2005
(73) Proprietor: Mega Disposables S.A., 13671 Aharnes (GR)
(72) Inventor: VITOULADITIS, Constantinos, GR-13671 Aharnes (GR)
(74) Representative: Kilimiris, Constantinos
(86) International application number: PCT/GR2003/000013
(87) International publication number: WO 2004/002387

(56) References cited:
- EP-A- 1 138 294
- US-A- 5 221 275
- US-A- 5 713 886

## Description

### FIELD OF THE INVENTION

The present invention relates to menstrual absorbent articles, such as female sanitary napkins, which are designed to be worn in and attached to thong slips or G-string undergarments.

Current fashion, with tight-fitting, figure-hugging clothes, has meant that it has become more common to wear so-called thongs, or G-string underwear, which have a very narrow crotch area.

In general absorbent articles for wearing with and attached to an undergarment should be designed to follow the shape of the underwear where they are attached. For a thong slip this results in a sanitary napkin having a wider front end and a narrower rear end.
It has been proposed to design the absorbent articles with a shape matching thong underwear.

In WO 97 / 39713 such an article is taught. Although such an article functions satisfactorily in terms of discretion, it has proven difficult to get the article to fit securely in place in the thong during use.

Thus, one of the most important issues to be solved in absorbent articles for said type of briefs, is the fastening to the briefs, since the crotch area of the thong is very small.

It is also important, for several reasons, for the article, to fit correctly in the thong. In fact, on one side, an incorrect positioning of the article means that the discretion and the comfort of the wearer suffers, while, on another side, it is particularly important that the article, on account of its small surface area, should be placed in such a way that it is able to take up and absorb all the body fluids eliminated.

Secure and correct fastening is therefore also important from point of view of leakage.
US Des. 394503 discloses an article with a shape which is intended to fit in a thong underwear and which is additionally provided with protruding side tabs.

Said tabs in this model have a rectangular shape with sharp edges, which will cause discomfort because said sharp edges will chafe or cut into the wearer during use.

US 5713886 describes panty liners intended to be worn in a thong and provided with fastening tabs. The fastening tabs are located both on the wider and on the narrower portions of the article.

The disadvantage of an article according to this patent is that the two tabs placed on the wider portion of the article reduce the adaptability to different shapes and sizes of thong. A further disadvantage is that the tabs on the wider portion will be visible during use, which takes away from advantages of wearing thongs.

WO 00/72790 teaches an absorbent article for use in thong underwear characterized in having two fastening tabs applied along the sides of the narrower portion of the article.

WO 01/78637 similarly teaches a sanitary napkin intended to be worn inside thong panties, said sanitary napkin including two outwardly projecting, flexible flaps, which extend along the tapering part of the article.

EP-A-1 138 294 teaches an absorbent article to be worn with a thong underwear having an anterior, a posterior and a midsection and two flaps (9a and 9b) which extend laterally from each side of the posterior portion.

Both of said two International Applications present a heavy limitation in teaching a configuration of the fastening tabs and of the projecting flaps which, during fixation on a thong panty, overlap each other, rendering very uncomfortable the separation of the article from the thong panty after use.

Said limitation drives to unaccepted risks of staining the hands of the wearer, if not even to the tearing of the article, to obtain its removal.

### SUMMARY OF THE INVENTION

It is therefore an object of the present invention to provide a system to secure the article in the thong in order to permit an easy removal after use.

It is another object of the present invention to provide a securing system that is not visible during use.

The absorbent article in accordance with this invention includes one single flap projecting outwardly along one of the sides of the narrower portion of the tapered article. Extension of the flap is such that, once folded along a thong panty, it does not exceed the width of the thong panty.

In order to enhance the non visibility during use, it is another object to provide a transparent flap, by means of properly selected materials included in the flap; the scope is to provide a flap such that its color does not create a highlight relative to the color of the undergarment in which it is worn.

WO 01/72252 teaches the arrangement of two transparent flaps, in order to achieve an at least partially invisible flaps.

Nevertheless it is well known that any material or combination of materials will remove a certain fraction of light, therefore the complete transparency cannot exist.

Using two flaps for securing a sanitary towel onto a thong underwear, means to overlap the two flaps one on the other, in this reducing even more the effective total transparency; by means of a fixation achieved with one transparent flap, total transparency is dramatically improved.

It is further to be understood that the present invention may be embodied in other specific forms and it is desired that the present embodiments be considered in all respects as illustrative and, therefore, not restrictive, reference being made to the appended claims rather than to the foregoing description to indicate the scope of the invention.

### FIGURES

- Fig. 1: is a perspective view of the sanitary towel of the present invention
- Fig. 2: is a cross-sectional view taken along lines A-A of Fig. 1
- Fig. 3: is a cross-sectional view of an alternative embodiment

### DETAILED TECHNICAL DESCRIPTION

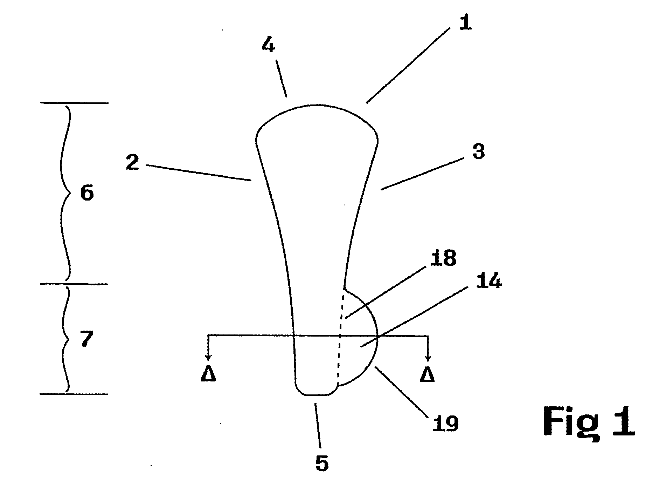

Fig. 1 shows a sanitary towel according to one embodiment of the present invention.

The sanitary towel 1 has an essentially elongate shape with a longitudinal direction and a transverse direction and it has two long sides 2, 3, two short sides 4, 5, a first end portion 6 and a second end portion 7.

The sanitary towel 1 has an upper side 10, intended to be directed towards the wearer during use, and an underside 20, intended to be directed away from the wearer during use.

The sanitary towel comprises a liquid-permeable surface layer 12 arranged on that side of the sanitary towel intended to be directed towards the wearer during use, called the upper side 10, and a liquid-barrier backing layer 22 arranged on that side of the sanitary towel intended to be directed away from wearer during use, called the underside 20.

Between the surface layer 12 and the backing layer 22 is included an absorbent body 30.

The surface layer 12 can be of any conventional material, for example a non-woven, an apertured plastic film or a laminate of an apertured plastic film with a non-woven.

The liquid barrier 22 or backing layer is made out of a liquid-impermeable material.
Thin, liquid-tight plastic films are suitable at this purpose, although it is possible to use a material which is originally liquid-permeable, that has been provided with a coating of plastic, resin or other liquid-tight material.

Examples of materials that can be used as barriers are plastic films, nonwovens and laminates of these. The plastic film can be, for example, polyethylene, polypropylene or polyester.

The absorbent core 30 is preferably made of cellulose pulp, either in the form of a de-fibered roll or bale (so said fluff) or in the form of a cellulose pulp based air laid non-woven.

The absorbent body can also include super-absorbent polymers or fibers, as well as stabilizing members, liquid-dispersing members, anti odor or antimicrobial agents. It is also possible to use absorbent foam materials in the absorbent body.

An acquisition layer 32 can be arranged between the surface layer 12 and the absorbent body 30.

The purpose of the acquisition layer 32 is to draw liquid into the sanitary towel and convey it down to the absorbent body 30. The acquisition layer 32 can be made of low-density non-woven materials.

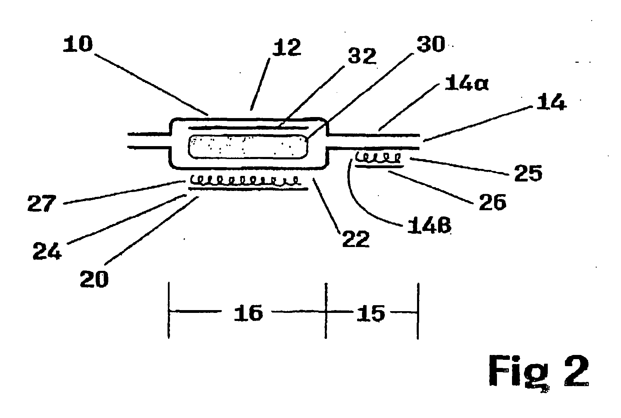

As it can be seen in Fig. 2, the attached portions of the surface layer 12 and the backing layer 22 are dimensioned to provide the sanitary towel with one side flap 14 , formed by an upper surface 14a and a lower surface 14b, capable of being folded around the narrowed crotch portion of an undergarment like a G String or thong.

Shape and size of said flap can be various; in a preferred embodiment the maximum width 15 of the flap 14 is not exceeding the width 16 of the sanitary napkin in correspondence to same transversal axis than the axis of the width 15, whereas the flap 14 is considered to be folded in use along a line 18 that is an axis of flexibility corresponding to juncture line of flap 14 with article 10, in this delimitating segments 15 and 16.

In another embodiment an axis of flexibility is provided along a line included between juncture line 18 and distal edge 19 of the flap; according to this embodiment, width 15, which will not exceed the width 16 of the sanitary napkin, is obtained as the distance between distal edge 19 and said intermediate axis of flexibility.

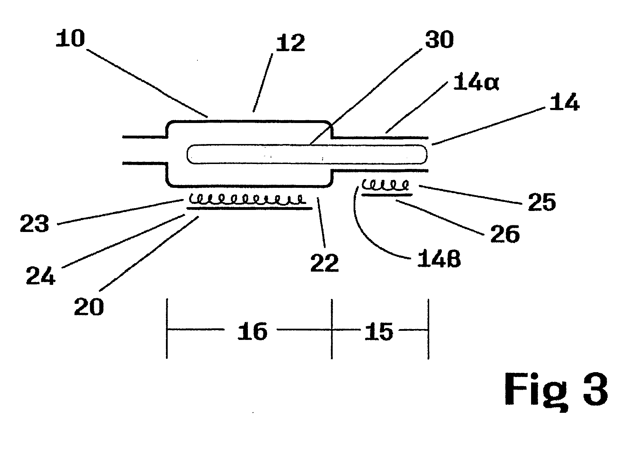

In another embodiment the absorbent body 30 and secured acquisition layer 32 can extend into the flap 14, being included between surfaces 14a and 14b. (Fig. 3)

Portions of adhesives 23 are arranged on the external surface of the backing layer 22, in order to permit an easy stay in place during use, by means of a fixation on the internal side of a thong underwear; said adhesives are covered from a release liner 24 which protects said adhesives 23 before use.

Portions of adhesives 23 can cover completely or only a limited area of surface 22.
Release liner 24 can cover completely or only a limited area of surface 22.

Portion of adhesives 25 are arranged on the external or lower side 14b of the flap 14, in order to permit the fixation of the flap 14, after having been folded preferably along the line 18, on the external side of a thong underwear; said portion of adhesives 25 is covered from a release patch 26.

Portions of adhesives 25 can cover completely or only a limited area of surface 14b.
Release patch 26 can cover completely or only a limited area of surface 14b.
Release patch 26 can be also a coextension of release liner 24.

In another embodiment of this invention; transparent materials can be used for the arrangement of a flap, obtained as a derivation from attached portions of the surface layer and of the backing layer.

The top sheet can be made from a non-woven or woven material or an apertured film; said non-woven, or woven or films are obtained from polymers which have been provided with a coloring material such as a titanium dioxide to provide a white opacity.

Simply omitting the coloring material provides a transparent film or non-woven, which has a high level of light transmission.

As with the top sheet the entire back sheet can be realized using materials during whose formation, the white color filler, as a titanium oxide, has been eliminated.

## Claims

1. An absorbent article, such as a sanitary towel or an incontinence protector, said article intended to be worn with a thong underwear, having an essentially elongate shape with a longitudinal direction and a transverse direction, an upper side (10) and an under side (20), two long sides (2) and (3), two short sides (4) and (5), a first end portion, intended to be directed forward on the user and a second portion (7), intended to be directed rearwards on the user where first portion is wider than the second end portion, a liquid-permeable surface layer, a liquid-barrier backing layer, an absorbent body included between surface layer and backing layer, **characterized in that** one single flap (14) extends laterally from only one of the two long sides, in the second end portion and no further flaps are provided in the first end portion and **in that** the maximum width (15) of the flap (14) does not exceed the width (16) of the absorbent article on same transversal axis.

2. An absorbent article according to claim 1, wherein the flap is provided as en extension of the attached portions of the surface layer and of the backing layer.

3. An absorbent article according to claims 1 and 2, where an axis of flexibility is provided along a line included between juncture line (18) and distal edge (19), in this determining a width (15) as distance between said distal edge and said juncture line.

4. An absorbent article according to claims 1, 2 and 3 where the absorbent core and secured acquisition layer (32) are extended into the flap (14)

5. An absorbent article according to claims 1, 2, 3, 4 wherein both the materials used for the surface layer and the backing layer are transparent, meaning that these materials are either non-woven, or woven or films, which have been realized having omitted any inclusion of color fillers.

## Patentansprüche

1. Absorbierender Artikel, wie etwa eine Monatsbinde oder eine Inkontinenzschutzeinrichtung, wobei der Artikel dafür gedacht ist, mit einem Tangaslip getragen zu werden, und aufweist: eine im wesentlichen längliche Form mit einer Längsrichtung und einer Querrichtung, eine Oberseite (10) und eine Unterseite (20), zwei lange Seiten (2) und (3), zwei kurze Seiten (4) und (5), einen ersten Endabschnitt, der dafür gedacht ist, am Benutzer nach vorne ausgerichtet zu sein, und einen zweiten Abschnitt (7), der dafür gedacht ist, am Benutzer nach hinten ausgerichtet zu sein, wobei der erste Abschnitt breiter als der zweite Endabschnitt ist, eine flüssigkeitsdurchlässige Oberflächenschicht, eine untere Flüssigkeitssperrschicht, einen absorbierenden Körper, der zwischen der Oberflächenschicht und der unteren Schicht angeordnet ist, **dadurch gekennzeichnet, daß** sich in dem zweiten Endabschnitt ein einziger Flügel (14) seitlich von nur einer der zwei langen Seiten erstreckt und keine weiteren Flügel in dem ersten Endabschnitt bereitgestellt sind und daß die maximale Breite (15) des Flügels (14) die Breite (16) des absorbierenden Artikels auf der gleichen Querachse nicht überschreitet.

2. Absorbierender Artikel nach Anspruch 1, wobei der Flügel als eine Erweiterung der angeschlossenen Abschnitte der Oberflächenschicht und der unteren Schicht bereitgestellt ist.

3. Absorbierender Artikel nach Anspruch 1 und 2, wobei eine Flexibilitätsachse entlang einer Linie, die zwischen der Verbindungslinie (18) und dem distalen Rand (19) enthalten ist, bereitgestellt ist, wobei eine Breite (15) als ein Abstand zwischen dem distalen Rand und der Verbindungslinie bestimmt wird.

4. Absorbierender Artikel nach Anspruch 1, 2 und 3, wobei der Absorptionskern und die befestigte Aufnahmeschicht (32) in den Flügel (14) ausgedehnt sind.

5. Absorbierender Artikel nach den Ansprüchen 1, 2, 3, 4, wobei die für die Oberflächenschicht und die untere Schicht verwendeten Materialien beide transparent sind, was bedeutet, daß diese Materialien entweder Vlies oder Gewebe oder Folien sind, die unter Weglassen jeglicher Einlagerung von Farbfüllstoffen realisiert wurden.

## Revendications

1. Article absorbant, tel qu'une serviette hygiénique ou une protection contre l'incontinence, ledit article destiné à être porté avec un string, ayant une forme essentiellement allongée avec une direction longitudinale et une direction transversale, un côté supérieur (10) et un côté inférieur (20), deux côtés longs (2) et (3), deux côtés courts (4) et (5), une première portion d'extrémité, destinée à être dirigée vers l'avant sur l'utilisateur et une deuxième portion (7) destinée à être dirigée vers l'arrière sur l'utilisateur où la première portion est plus large que la deuxième portion d'extrémité, une couche de surface perméable aux liquides, une couche postérieure faisant barrière aux liquides, un corps absorbant inclus entre la couche de surface et la couche postérieure, **caractérisé en qu'**un rabat unique (14) s'étend latéralement à partir d'un seul des deux côtés longs, dans la deuxième portion d'extrémité et qu'aucun autre rabat n'est prévu dans la première portion d'extrémité et en ce que la largeur maximum (15) du rabat (14) n'excède pas la largeur (16) de l'article absorbant sur le même axe transversal.

2. Article absorbant selon la revendication 1, dans lequel le rabat est prévu comme une extension des portions attachées de la couche de surface et de la couche postérieure.

3. Article absorbant selon les revendications 1 et 2, où un axe de flexibilité est prévu le long d'une ligne incluse entre une ligne de jonction (18) et un bord distal (19), déterminant en cela une largeur (15) comme distance entre ledit bord distal et ladite ligne de jonction.

4. Article absorbant selon les revendications 1, 2 et 3, où le coeur absorbant et la couche d'acquisition sécurisée (32) sont étendus dans le rabat (14).

5. Article absorbant selon les revendications 1, 2, 3 et 4 dans lequel les matériaux utilisés pour la couche de surface et la couche postérieure sont tous les deux transparents, ce qui signifie que ces matériaux sont soit non tissés, soit tissés, soit des films, qui ont été réalisés en ayant omis toute inclusion de charges de couleur.
